(19) Europäisches Patentamt
European Patent Office
Office européen des brevets

(11) Publication number: 0 197 386 B1

# (12) EUROPEAN PATENT SPECIFICATION

(45) Date of publication of the patent specification: 02.11.88

(51) Int. Cl.⁴: C 07 C 143/78, C 07 C 143/822, C 07 D 295/08, C 07 D 241/08, A 61 K 31/19, A 61 K 31/24

(21) Application number: 86103792.7

(22) Date of filling: 20.03.86

## (54) 2-(Substituted sulfamyl) derivatives of 6-nitrobenzoic acid, process for their preparation and pharmaceutical compositions containing them.

(30) Priority: 27.03.85 US 716886
06.11.85 US 795569
06.11.85 US 795564

(43) Date of publication of application:
15.10.86 Bulletin 86/42

(45) Publication of the grant of the patent:
02.11.88 Bulletin 88/44

(84) Designated Contracting States:
CH DE FR GB IT LI NL

(56) References cited:
EP-A-0 068 407
EP-A-0 068 408
DE-C-551 423
FR-A-2 493 702

CHEMICAL ABSTRACTS, vol. 62, no. 9, 26th April 1965, column 10362 e-g, Columbus, Ohio, US; G.H. HAMOR et al.: "Synthesis of alkyl esters of 4-amino-2-sulfamoylbenzoic acid"

(73) Proprietor: MERCK & CO. INC., 126, East Lincoln Avenue P.O. Box 2000, Rahway New Jersey 07065- 0900 (US)

(72) Inventor: Saari, Walfred S., 1740 Wagon Wheel Lane, Lansdale, PA 19446 (US)
Inventor: Engelhardt, Edward L., 904 Plymouth Road, Gwynedd Valley, PA 19437 (US)

(74) Representative: Blum, Rudolf Emil Ernst, c/o E. Blum & Co Patentanwälte Vorderberg 11, CH-8044 Zürich (CH)

EP 0 197 386 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

LIBER, STOCKHOLM 1988

**Description**

At the present time, certain other unrelated compounds are in experimental clinical use as radiation sensitizers. However, these compounds - for example, metronidazole and misonidazole - suffer from the drawback that they also cause neurotoxicity which limits their usefulness.

In Chemical Abstracts, vol. 62, no. 9, 26th April 1965, column 10362, there is described the synthesis of alkyl esters of 4-amino-2-sulfamoyl benzoic acids. One starting material disclosed in said abstracts is the nitro ester having the following formula

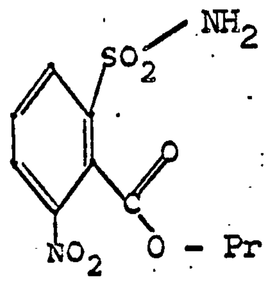

wherein Pr is the isopropyl residue. No pharmaceutical activity is stated for said starting material.

The compounds of the present invention are effective radiation sensitizers, and are believed to have a more favorable therapeutic ratio.

**Detailed description of the invention**

The compositions of the present invention are nitrobenzenesulfonamide compounds of the formula

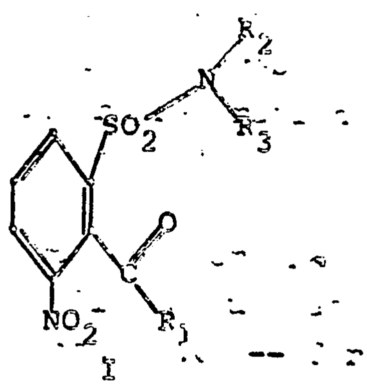

I

wherein

$R_1$ is hydroxy, hydroxy-(lower alkoxy), lower alkoxy, allyloxy, amino, monoalkylamino, dialkylamino, hydroxyalkylamino, di-(hydroxyalkyl)-amino, or allylamino,

$R_2$ and $R_3$ are each separately hydrogen, lower alkyl from 1 - 4 carbon atoms, hydroxy-(lower alkyl), allyl, amino-(lower alkyl), (lower alkyl)-amino-(lower alkyl), di-(lower alkyl)-amino-(lower alkyl), (hydroxyalkyl)-amino-(lower alkyl), (hydroxyalkyl)-alkylamino-(lower alkyl) or di-(hydroxyalkyl)-amino-(lower alkyl) or when taken together along with the nitrogen to which they are attached represent a heterocyclic ring selected from morpholino or $R_4$-substituted-3-oxo-piperazin-1-yl

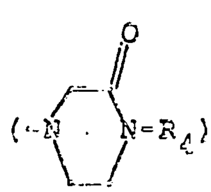

wherein $R_4$ is hydrogen, lower alkyl of from 1 - 4 carbons, or hydroxyalkyl of from 1 - 4 carbons,
provided that if both radicals $R_2$ and $R_3$ have the meaning of a hydrogen atom, then $R_1$ must not be an alkoxy group and
salts of the compounds of formula I.

The 2-(substituted sulfamyl) derivatives of 6-nitrobenzoic acid, ester and amide compounds of the present invention are prepared in the following manner:

A substituted nitrobenzoate ester or nitrobenzamide having a 2-chlorosulfonyl substituent in an aprotic solvent such as tetrahydrofuran, dioxane, dimethoxyethane, or chloroform is treated with at least an equimolar amount of an amine of the formula

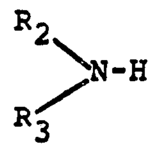 II

wherein $R_2$ and $R_3$ are as described hereinabove.

It is preferred to carry out the reaction in the presence of a base in sufficient amount to neutralize the hydrogen chloride formed in the course of the reaction. The base utilized may be a tertiary amine such as triethylamine or pyridine. On the other hand the same results may be produced by adding at least twice the molar amount of reactant amine theoretically required. In this event, the reactant amine is utilized both to form the sulfonamide and to neutralize the hydrogen chloride formed in the amination reaction.

The temperature at which the reaction is carried out is not critical and may vary from 0°-100°C or at the reflux temperature of the solvent, if under 100°C. The reaction temperature is preferably maintained at about 0 - 25°C for a period of 1 - 24 hours. The amination reaction may be formulated as follows:

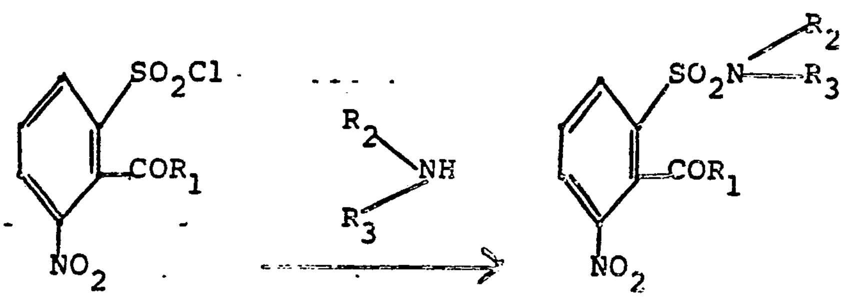

wherein $R_1$, $R_2$ and $R_3$ are as defined hereinabove.

The starting materials for the process are either known or are readily prepared from the known 2-amino-6-nitrobenzoic acid by a process of esterification followed by diazotization of the amino group and treating the formed diazonium compound with $SO_2$ in the presence of $CuCl_2$ whereby the desired starting 2-chlorosulfonyl-6-nitrobenzoate ester is formed.

The ester derivatives of this invention may also be prepared by esterification of a carboxylic acid of formula I ($R_1 = OH$). Established methods for the esterification of carboxylic acids containing basic groups may be used. These include reaction with diazoalkanes or with alcohols under strongly acidic conditions.

The benzamide derivatives of this invention may be prepared by reaction of a 2-monosubstituted sulfamyl-6-nitrobenzoate ester of formula III or a 2-substituted-4-nitro-2H-1,2-benzisothiazol-3-one 1,1-dioxide of formula IV with at least one equivalent of ammonia or a mono- or dialkylsubstituted amine of formula II.

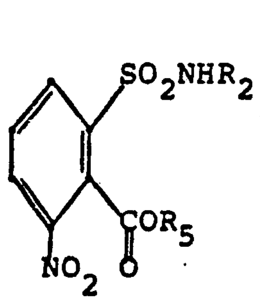

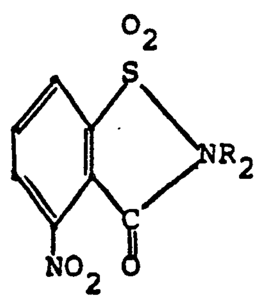

III IV

In formulas III and IV, $R_2$ is as described hereinabove and $R_5$ is either lower alkyl or hydroxy-(lower alkyl). The reaction is carried out in a suitable solvent such as a lower aliphatic alcohol or a polar aprotic solvent such as dimethylformamide, dimethylsulfoxide or others such as tetrahydrofuran, glyme, diglyme, chloroform or methylenechloride. The reaction temperature is not critical and may vary from 0 - 100°C, preferably from about

25° - 50°C for a period of 1 to 10 days. When low boiling amines are used, the reaction may be run in a sealed vessel.

The compounds of this invention may also be prepared by alkylation of an amine with an amide or ester of formula V wherein $R_1$ and $R_2$ are as defined hereinabove and $R_6$ is an alkylating moiety such as haloalkyl, alkylsulfonyloxyalkyl or arylsulfonyloxyalkyl.

The reaction is carried out in a suitable aprotic solvent such as dimethylformamide, acetonitrile or the like. The reaction temperature may vary from 50°C to the boiling point of the solvent for a period of 1 to 10 days. When low boiling amines are used, the reaction may be run in a sealed vessel. It is preferred to carry out the reaction in the presence of a base in sufficient amount to neutralize the acid formed in the course of the reaction. The base utilized may be a tertiary amine such as a trialkylamine or pyridine. Alternatively, at least twice the molar amount of reactant amine theoretically required may be used. In this event, the reactant amine is utilized both to form the desired product end to neutralize the acid formed in the amination reaction.

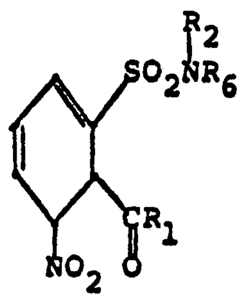

V

The alkylating agents of formule V are readily prepared from the corresponding alcohols by established methods.

The method of treatment of human patients or domestic animals undergoing radiation treatment of malignant disease processes employs the compounds of the present invention in pharmaceutical compositions that are administered orally or intravenously. The dose employed depends on the radiation protocol for each individual patient. In protocols where the radiation dose is divided into a large number of fractions, the drug can be administered at intervals in the schedule and not necessarily with each radiation treatment. It should be noted that the compounds of the present invention are not intended for chronic administration. In general, the drug is administered from 10 minutes to 5 hours prior to the radiation treatment in a dosage amount of between 0.25 to about 4.0 grams per square meter of body surface.

The dosage range given is the effective dosage range and the decision as to the exact dosage used must be made by the administering physician based on his judgement of the patient's general physical condition. In determining the dose for the individual patient, the physician may begin with an initial dose of 0.25 g/square meter of body surface to determine how well the drug is tolerated and increase the dosage with each succeeding radiation treatment, observing the patient carefully for any drug side effect. The composition to be administered is an effective amount of the active compound and a pharmaceutical carrier for said active compound.

The dosage form for intravenous administration is a sterile isotonic solution of the drug. Oral dosage forms such as tablets, capsules, or elixirs may also be used.

Capsules or tablets containing 25, 50, 100 or 500 mg of drug/capsule or tablets are satisfactory for use in the method of treatment of our invention.

The following examples are intended to illustrate but do not limit the process of preparation, product, compositions, or method of treatment aspects of the invention. Temperatures are in degrees Celsius unless otherwise indicated throughout the application.

**Examples**

Part A - Examples of compounds in which at least one of $R_2$ and $R_3$ is a basic substituent.

**Example 1**

Step A: Methyl 2-Amino-6-nitrobenzoate

A mixture of 2-amino-6-nitrobenzoic acid (11.9 g, 65.3 mmol), methyl p-toluenesulfonate (15.1 g, 81.1 mmol) and triethylamine (6.60 g, 65.3 mmol) in DMF (170 ml) was stirred under $N_2$ at 60° for 18 hours. After removing

DMF at 60° and 0.2 mm pressure, the residue was dissolved in ETOAc and washed with a saturated solution of $NaHCO_3$ followed by a saturated aqueous solution of NaCl. The EtOAc extract was dried ($Na_2SO_4$), filtered and concentrated under reduced pressure. Flash chromatography over silica gel and elution with 50 % toluene-50 % $CHCl_3$ gave methyl 2-amino-6-nitro-benzoate (7.6 g, 59.4 %), m.p. 105 - 107°.

Step B: Methyl 2-Chlorosulfonyl-6-nitrobenzoate

To a suspension of methyl 2-amino-6-nitrobenzoate (7.6 g, 38.7 mmol) in glacial acetic acid (37 ml) and conc. HCl (67 ml), cooled to - 5°, was added slowly a solution of sodium nitrite (2.86 g, 41,4 mmol) in $H_2O$ (11.2 ml). After addition was complete, the mixture was stirred at - 5° to 0° for an additional 30 minutes. During this time, a solution of $CuCl_2.2H_2$ (2.45 g) in $H_2O$ (8.5 ml) was prepared and added to a cold solution of $SO_2$ (25 g, 0.39 mol) in glacial acetic acid (50 ml). The diazonium salt solution was then added in portions to the cooled $SO_2$-$CuCl_2$ mixture. After stirring in an ice bath for 3 hours, the reaction mixture was allowed to warm to 20 - 25° and was stirred at this temperature for 18 hours. The reaction mixture was then poured onto ice (500 g), the precipitated tan solid removed by filtration and dried to give the sulfonyl chloride (9.1 g, 84.3 %), m.p. 152 - 4°.

**Example 2**

Methyl 2-[N-(2-Dimethylaminoethyl)-aminosulfonyl]-6-nitrobenzoate Hydrochloride and 2-[N-(2-Dimethylaminoethyl)-aminosulfonyl]-6-nitrobenzoic acid

A solution of methyl 2-chlorosulfonyl-6-nitrobenzoate (500 mg, 1.79 mmol) and 2-dimethylaminoethylamine (316 mg, 3.58 mmol) in THF (35 ml) was stirred in an ice bath for 1 hour then at 20 - 25° for 18 hours. After removing THF under reduced pressure, the residue was partitioned between saturated $Na_2CO_3$ solution and EtOAc. The organic extract was washed (saturated NaCl solution), dried ($Na_2SO_4$), filtered and concentrated under reduced pressure. Chromatography over silica gel (elution with 5 % MeOH-95 % $CHCl_3$) gave an oil which when triturated with EtOAc afforded 2-[N-(2-dimethylaminoethyl)-aminosulfonyl]-6-nitrobenzoic acid (90 mg, 16 %), m.p. 193° decomposed with effervescence.

Treatment of the EtOAc soluble fraction with anhydrous EtOH-HCl and recrystallization from EtOH-EtOAc gave the HCl salt of the méthylester (250 mg, 38 %), m.p. 170 - 173°.

**Example 3**

N,N-Dimethyl-2-[N-(2-dimethylaminoethyl)-aminosulfonyl]-6-nitrobenzamide Hydrochloride

A solution of methyl 2-[N-(2-dimethylaminoethyl)-aminosulfonyl]-6-nitrobenzoate (200 mg, 0.544 mmol) and 0.5 ml of a 40 % aqueous dimethylamine solution in methanol (5 ml) was allowed to stand at 20 - 25° for 18 hours. After concentrating under reduced pressure, the residue was treated with anhydrous ethanolic hydrogen chloride and recrystallized from MeOH-EtOAc to give the HCl salt, m.p. 166 - 69°.

**Example 4**

N,N-Dimethyl 2-[N-(2-Dimethylaminoethyl)-N-(2-hydroxyethyl)-aminosulfonyl]-6-nitrobenzamide Hydrochloride

Step A: N,N-Dimethyl 2-[N-(2-dimethylaminoethyl)-N-(2-(2-tetrahydro-2H-pyranyloxy)-ethyl)-aminosulfonyl]-6-nitrobenzamide

To a suspension of N,N-dimethyl 2-[N-(2-dimethylaminoethyl)-aminosulfonyl]-6-nitrobenzamide hydrochloride (347 mg, 0.91 mmol) in DMF (5 ml) under $N_2$ was added 50 % NaH (87 mg, 1.82 mmol). After stirring at 20 - 25° for 15 minutes until all of the NaH had reacted, a solution of 2-(2-bromoethoxy)-tetrahydro-2H-pyran in DMF (2 ml) was added. The solution was stirred at 20 - 25° under $N_2$ for 20 hours and then concentrated under reduced pressure to remove most of the DMF. The residue was partitioned between EtOAc and a saturated aqueous solution of NaCl. After drying ($Na_2SO_4$) the EtOAc extract, filtering and concentrating, the residue was chromatographed over silica gel. Elution with 7 % MeOH-93 % $CHCl_3$ gave 100 mg of product.

Step B: N,N-Dimethyl 2-[N-(2-dimethylaminoethyl)-N-(2-hydroxyethyl)-aminosulfonyl]-6-nitrobenzamide Hydrochloride

A solution of the tetrahydropyranyl ether (100 mg) in THF (10 ml), $H_2O$ (5 ml) and HOAc (20 ml) was stirred at 50° for 24 hours. After concentrating under reduced pressure, the residue was partitioned between EtOAc and saturated $NaHCO_3$ solution. The EtOAc extract was washed (saturated NaCl solution), dried ($Na_2SO_4$), filtered and concentrated. The crude product was purified by conversion to the HCl salt and recrystallized (MeOH-EtOAc-hexane) to give 32 mg of product, m.p. 162 - 64° C dec.

**Example 5**

N,N-Dimethyl 2- N-[2-(N-(2-Hydroxyethyl)-N-methylamino)-ethyl]-N-methylaminosulfonyl -6-nitrobenzamide Hydrogen Oxalate

Step A: N,N-Dimethyl 2-[N-(2-Methylsulfonyloxyethyl)-N-methylaminosulfonyl]-6-nitrobenzamide

A solution of N,N-dimethyl 2-[N-(2-hydroxyethyl)-N-methylaminosulfonyl]-8-nitrobenzamide, prepared as described in Example 9 USSN Docket No. Case 17223IB of Walfred Saari filed on the same date as the present case and incorporated herein by reference, (1.0 g, 3.0 mmol) and methanesulfonyl chloride (0.71 g, 6.2 mmol) in pyridine (10 ml) was stirred at 20 - 25° for one day. After concentrating under reduced pressure, the residue was partitioned between ethyl acetate and IN aqueous HCl. The ethyl acetate extract was washed with water, dried and concentrated. Pure mesylate (1.0 g) was obtained by flash chromatography over silica gel and elution with a 1 % methanol-99 % chloroform solvent mixture.

Step B: N,N-Dimethyl 2- N-[2-(N-(2-Hydroxyethyl)-N-methylamino)-ethyl]-N-methylaminosulfonyl-6-nitrobenzamide Hydrogen Oxalate

A solution of N,N-dimethyl 2-[N-(2-methylsulfonyloxyethyl)-N-methylaminosulfonyl]-6-nitrobenzamide (0.50 g, 1.2 mmol) and 2-(methylamino)-ethanol (0.22 g, 2.9 mmol) in acetonitrile (20 ml) was stirred at reflux for 20 hours. After concentrating under reduced pressure, the residue was partitioned between ethyl acetate and water. The ethyl acetate layer was dried ($Na_2SO_4$), filtered and concentrated. Flash chromatography of the residue over silica gel and elution with 5 % methanol-95 % chloroform gave 400 mg of pure product as an oil. The hydrogen oxalate salt, m.p. 159.0 - 162.0°, was prepared for analysis.

**Examples**

Part B - Examples in which each of $R_2$ and $R_3$ is a non-basic substituent.

**Example 1**

Methyl 2-[N-(2-Hydroxyethyl)-N-methylaminosulfonyl]-6-nitrobenzoate

Step A: Methyl 2-Amino-6-nitrobenzoate

A mixture of 2-amino-6-nitrobenzoic acid (11.9 g 65.3 mmol), methyl p-toluenesulfonate (15.1 g, 81.1 mmol) and triethylamine (6.60 g, 65.3 mmol) in DMF (170 ml) was stirred under $N_2$ at 60° for 18 hours. After removing DMF at 60° and 0.2 mm pressure, the residue was dissolved in EtOAc and washed with a saturated solution of $NaHCO_3$ followed by a saturated aqueous solution of NaCl. The EtOAc extract was dried ($Na_2SO_4$), filtered and concentrated under reduced pressure. Flash chromatography over silica gel and elution with 50 % toluene-50 % $CHCl_3$ gave methyl 2-amino-6-nitro-benzoate (7.6 g, 59.4 %), m.p. 105 - 107°.

Step B: Methyl 2-Chlorosulfonyl-6-nitrobenzoate

To a suspension of methyl 2-amino-6-nitrobenzoate (7.6 g, 38.7 mmol) in glacial acetic acid (37 ml) and conc. HCl (67 ml), cooled to - 5°, was added slowly a solution of sodium nitrite (2.86 g, 41,4 mmol) in $H_2O$ (11.2 ml). After addition was complete, the mixture was stirred at - 5° to 0° for an additional 30 minutes. During this time, a solution of $CuCl_2.2H_2O$ (2.45 g) in $H_2O$ (8.5 ml) was prepared and added to a cold solution of $SO_2$ (25 g, 0.39 mol) in glacial acetic acid (50 ml). The diazonium salt solution was then added in portions to the cooled $SO_2$-$CuCl_2$ mixture. After stirring in an ice bath for 3 hours, the reaction mixture was allowed to warm to 20 - 25° and was stirred at this temperature for 18 hours. The reaction mixture was then poured onto ice (500 g), the precipitated tan solid removed by filtration and dried to give the sulfonyl chloride (9.1 g, 84.3 %), m.p. 152 - 4°.

Step C: Methyl 2-[N-(2-Hydroxyethyl)-N-methyl-aminosulfonyl]-6-nitrobenzoate

N-Methylethanolamine (2.96 g, 39.4 mmol) was added to a solution of methyl 2-chlorosulfonyl-6-nitrobenzoate (5.5 g, 19.7 mmol) in THF (150 ml) and the mixture stirred at 20 - 25° for 18 hours. After removing THF under reduced pressure, the residue was partitioned between EtOAc and $H_2O$. The organic extract was washed with saturated NaCl solution and dried ($Na_2SO_4$). Flash chromatography of the residue over silica gel and elution with 1 % MeOH-99 % $CHCl_3$ afforded pure sulfonamide. Recrystallization from EtOAc-hexane gave analytically pure product (5.2 g, 82.9 %), m.p. 98 - 101°.

**Example 2**

Methyl 2-[N,N-Di-(2-hydroxyethyl)-aminosulfonyl]-6-nitrobenzoate

A solution of di-(2-hydroxyethyl)-amine (0.76 g, 7.2 mmol) in THF (10 ml) was added to a solution of methyl 2-chlorosulfonyl-6-nitrobenzoate (1.0 g, 3.6 mmol) in THF (10 ml) and the mixture stirred at 20 - 25° for 18 hours. After removing THF under reduced pressure, the crude product was extracted into EtOAc which was then washed ($H_2O$), dried ($Na_2SO_4$), filtered and concentrated under reduced pressure. Flash chromatography of the residue over silica gel and elution with 5 % MeOH-95 % $CHCl_3$ gave pure sulfonamide. Analytically pure material (0.56 g, 44.8 %), m.p. 92 - 3°, was obtained upon recrystallization from EtOAc-hexane.

**Example 3**

Methyl 6-Nitro-2-[3-oxo-1-piperazinylsulfonyl]-benzoate

Piperazin-2-one (0.36 g, 3.6 mmol) was added to a mixture of methyl 2-chlorosulfonyl-6-nitro-benzoate (1.0 g, 3.6 mmol) and triethylamine (0.37 g, 3.6 mmol) in $CHCl_3$ (120 ml) and the resulting solution stirred at 20 - 25° for 18 hours. Removal of $CHCl_3$ under reduced pressure and flash chromatography of the residue over silica gel (elution with 5 % MeOH-95 % $CHCl_3$) afforded pure sulfonamide (1.2 g, 96.8 %). Recrystallization from MeOH-$H_2O$ gave an analytical sample, m.p. 189 - 91°.

**Example 4**

Methyl 2-[N-Morpholinosulfonyl]-6-nitrobenzoate

A solution of morpholine (1.25 g, 14.3 mmol) in THF (20 ml) was added over 30 minutes to a stirred, cooled solution of methyl 2-chlorosulfonyl-6-nitrobenzoate (2.0 g, 7.15 mmol) in THF (20 ml). After stirring at 20 - 25° for 18 hours, THF was removed under reduced pressure. The residue was partitioned between EtOAc and saturated NaCl-$H_2O$ and the EtOAc extract was washed with $H_2O$, dried ($Na_2SO_4$), filtered and concentrated. Recrystallization from MeOH-EtOH gave pure sulfonamide (1.7 g, 72 %), m.p. 145 - 8°.

**Example 5**

Methyl 2-[N-(2-Hydroxyethyl)-aminosulfonyl]-6-nitrobenzoate

A solution of methyl 2-chlorosulfonyl-6-nitrobenzoate (0.50 g, 1.79 mmol) and ethanolamine (0.33 g, 5.4 mmol) in THF (20 ml) was stirred at 20 - 25° for 18 hours and then concentrated under reduced pressure. Product was extracted into EtOAc which was then washed ($H_2O$), dried ($Na_2SO_4$), filtered and concentrated under reduced pressure. The residue was recrystallized from EtOAc-hexane to give pure sulfonamide (0.28 g, 51.4 %), m.p. 110 - 12°.

**Example 6**

N-(2-Hydroxyethyl)-2-[N-(2-hydroxyethyl)-aminosulfonyl]-6-nitrobenzamide

Step A: 2-(2-Hydroxyethyl)-4-nitro-2H-1,2-benzisothiazol-3-one 1,1-dioxide

To approximately 2 ml of ethylene oxide cooled in an ice bath was added a suspension of 4-nitro-2H-1,2-benzisothiazol-3-one 1,1-dioxide (2.0 g, 8.76 mmol) in $H_2O$ (140 ml). After stirring in the ice bath for 1 hour, the mixture was allowed to stir at 20 - 25° for 18 hours. Water was removed under reduced pressure and the residue flash chromatographed over silica gel. Elution with 2 % isopropanol -98 % $CH_2Cl_2$ gave product which was recrystallized from EtOAc-hexane to give the 2-hydroxyethyl derivative (0.39 g, 16 %), m.p. 140 - 1°.

Step B: N-(2-Hydroxyethyl)-2-[N-(2-hydroxyethyl)-aminosulfonyl]-8-nitrobenzamide

A solution of 2-(2-hydroxyethyl)-4-nitro-2H-1,2-benzisothiazol-3-one 1,1-dioxide (100 mg, 0.37 mmol) and ethanolamine (24 mg, 0.39 mmol) in THF (5 ml) was allowed to stand at 20 - 25° for 3 days. After removal of THF under reduced pressure, the residue was recrystallized from MeOH-EtOAc-hexane to give 112 mg (91 %) of product, m.p. 176.5 - 177.5°.

**Example 7**

Allyl 2-[N-Morpholinosulfonyl]-6-nitrobenzoate

Step A: Allyl 2-Amino-6-nitrobenzoate

A mixture of 2-amino-6-nitrobenzoic acid (2.0 g, 11 mmol), allyl chloride (1.05 g, 13.7 mmol) and triethylamine (1.11 g, 11 mmol) in DMF (50 ml) was stirred at 60° for 18 hours. After concentrating under reduced pressure, the residue was extracted with EtOAc which was then washed with saturated $NaHCO_3$ solution and saturated NaCl solution, dried ($Na_2SO_4$) and filtered. EtOAc was removed under reduced pressure and the residue chromatographed over silica gel. Elution with 50 % hexane-50 % $CHCl_3$ gave the allyl ester (1.1 g, 45 %). An analytical sample, m.p. 54 - 5°, was obtained upon recrystallization from toluene-hexane.

Step B: Allyl 2-Chlorosulfonyl-6-nitrobenzoate

To a suspension of allyl 2-amino-6-nitrobenzoate (3.3 g, 14.9 mmol) in glacial acetic acid (80 ml) and conc. HCl (26 ml) cooled to - 5° was added slowly a solution of sodium nitrite (1.10 g 15.9 mmol) in $H_2O$ (6 ml). After addition was complete, the mixture was stirred at - 5° to 0° for an additional 30 minutes. This diazonium salt solution was then added in portions to a cold solution of $SO_2$ (10 g, 0.156 mol) and $CuCl_2.2H_2O$ (1.19 g) in acetic acid (20 ml) and $H_2O$ (4 ml). After stirring in an ice bath for 3 hours, the reaction mixture was allowed to warm to 20 - 25° and then poured on to ice (500 g). The solid sulfonyl chloride was filtered off and dried to give 3.5 g (77.4 %) of product, m.p. 68 - 70°. An analytical sample, m.p. 70 - 72°, was obtained upon recrystallization from n-butyl chloride-hexane.

Step C: Allyl 2-[N-Morpholinosulfonyl]-6-nitrobenzoate

A solution of morpholine (1.83 g, 18.7 mmol) in THF (30 ml) was added over 25 minutes to a stirred and cooled solution of allyl 2-chlorosulfonyl-6-nitrobenzoate (2.85 g, 9.3 mmol) in THF (80 ml). After stirring at 20 - 25° for 18 hours, THF was removed under reduced pressure and the residue partitioned between EtOAc and saturated NaCl solution. The EtOAc extract was dried ($Na_2SO_4$), filtered and concentrated to give a quantitative yield of the sulfonamide, m.p. 145 - 7°. An analytical sample, m.p. 150 - 2° was obtained upon recrystallization from MeOH.

**Example 8**

N,N-Dimethyl 2-[N-(2-hydroxyethyl)-aminosulfonyl]-6-nitrobenzamide

A solution of methyl 2-[N-(2-hydroxyethyl)-aminosulfonyl]-6-nitrobenzoate (4.58 g, 15.1 mmol) in absolute MeOH (50 ml) was added to a solution of dimethylamine (8.8 g, 0.15 mol) and potassium tertbutoxide (0.68 ml of a 0.282 M solution in tertbutanol) in absolute MeOH (100 ml). After stirring at 20 - 25°C for 4 days, solvents were removed under reduced pressure. The residue was dissolved in EtOAc and washed with 0.5N HCl followed by a saturated aqueous NaCl solution. The EtOAc extract was dried ($Na_2SO_4$), filtered and concentrated to give 4.2 g (88 %) of product.

**Example 9**

N,N-Dimethyl 2-[N-(2-hydroxyethyl)-N-methylaminosulfonyl]-6-nitrobenzamide

A solution of N,N-Dimethyl 2-[N-(2-hydroxyethyl)-aminosulfonyl]-6-nitrobenzamide (3.97 g, 12.5 mmol) in dry DMF (40 ml) was added slowly to a stirred suspension of 50 % NaH (0.60 g, 12.5 mmol) in dry DMF (10 ml) under $N_2$ at 20 - 25°C. After formation of the sodium salt was complete, a solution of methyl p-toluenesulfonate (2.40 g, 12.9 mmol) in DMF (4 ml) was added and the reaction mixture stirred at 20 - 25°C for 20 hours and then at 60°C for 23 hours. The orange solution was mixed with EtOAc (400 ml) and the white solid which formed was filtered off. This precipitate was washed with EtOAc (100 ml). The combined EtOAc solutions were washed with a saturated aqueous NaCl solution, dried ($Na_2SO_4$), filtered and concentrated under reduced pressure. Pure product, m.p. 107 - 108°C, 2.5 g (60 %) was obtained by flash chromatography over silica gel and elution with a 65 % n-butylchloride-35 % acetonitrile solvent mixture.

**Example 10**

Methyl 2-[N-(3-Hydroxypropyl)-aminosulfonyl]-6-nitrobenzoate

A solution of methyl 2-chlorosulfonyl-6-nitrobenzoate (2.80 g, 10 mmol) in 100 ml of THF was cooled to ice temperature and stirred in an ice-bath while a solution of 3-amino-1-propanol (99 %) (1.67 g, 22 mmol) was added dropwise over a period of 45 minutes. Stirring in the ice-bath was continued for 30 minutes. The reaction

mixture then was acidified by addition of 3.0 ml of 1.2N HCl. The THF was evaporated under reduced pressure and the residue taken up in 100 ml of ethyl acetate. After extracting this solution with 4 x 20 ml of saturated NaCl solution, the ethyl acetate was evaporated and the residue recrystallized from a mixture of ethyl acetate and hexane to give 2.70 g (84.9 %) of light yellow crystalline product, m.p. 87 - 88.5°.

**Example 11**

Methyl 2-[N-(2-Hydroxy-1-propyl)-aminosulfonyl]-6-nitrobenzoate

A solution of methyl 2-chlorosulfonyl-6-nitrobenzoate (2.80 g, 10 mmol) in 100 ml of THF was cooled in an ice-bath and stirred while a solution of 1-amino-2-propanol (1.65 g, 22 mmol) in 10 ml of THF was added dropwise over a period of 50 minutes. After stirring an additional 30 minutes in the ice-bath, the reaction mixture was acidified by the addition of 3 ml of 1.2N HCl. The THF was evaporated under reduced pressure and the residue taken up in 100 ml of ethyl acetate. After extracting with 4 x 20 ml of saturated NaCl solution, the ethyl acetate solution was dried over $Na_2SO_4$ and the solvent evaporated. The residue was crystallized from a mixture of ethyl acetate and hexane to give 2.02 g (63.5 %) of yellow crystalline product, m.p. 93.5 - 95°.

**Example 12**

N,N-Dimethyl-2-[N-(3-hydroxypropyl)-aminosulfonyl]-6-nitrobenzamide

Methyl-2-[N-(3-hydroxypropyl)-amino sulfonyl]-6-nitrobenzoate (2.00 g, 6.28 mmol) was dissolved in 60 ml. of methanol. Sodium methoxide (1.0 ml of a 0.1M solution in methanol) was added and the solution cooled in an ice-bath and stirred while a rapid stream of dimethylamine was passed into the vortex for 15 minutes. After stirring for 16-1/2 hours, during which time the temperature rose to 25°, the methanol and excess dimethylamine were evaporated under reduced pressure. Flash chromatography of the residue on E. Merck silica gel 60 (230 - 400 mesh) developed with n-butyl chloride : acetonitrile in the ratio of 65 : 35 gave light yellow crystalline product, isolated in two fractions, each melting at 85 - 87°. The second fraction contained a small amount of a second component, visible on TLC. Recrystallization of each fraction from a mixture of ethyl acetate and hexane gave 0.48 g of product, m.p. 86 - 87.5° and 0.43 g of product, m.p. 85.5 - 87°. Each product was analytically pure.

**Example 13**

N,N-Dimethyl-2-[N-(2-Hydroxy-1-propyl)-aminosulfonyl]-6-nitrobenzamide

Methyl 2-[N-(2-hydroxy-1-propyl)-amino-sulfonyl]-6-nitrobenzoate (2.00 g, 628 mmol) was dissolved in 60 ml of methanol. Sodium methoxide (1.0 ml of a 0.1M solution in methanol) was added and the solution cooled and stirred in an ice-bath while a rapid stream of dimethylamine was passed into the vortex for 15 minutes. Stirring in the ice-bath then was continued for 1 hour, when the flash was removed from the bath and the reaction mixture allowed to stand at room temperature overnight. The methanol and excess dimethylamine then were evaporated under reduced pressure and the residue shaken with ethyl acetate, 80 ml; 0.2N HCl, 10 ml; and saturated NaCl, 10 ml. The ethyl acetate layer was separated, extracted with 3 x 15 ml of saturated NaCl and evaporated to give a clear yellow oil. After drying 23 hours in vacuo, this product weights 2.03 g. Crystallization had started after 3 days. Recrystallization from a mixture of ethyl acetate and hexane gave 1.33 g (63.9 %) of product, m.p., 95.5 - 97°.

**Example 14**

N,N-Dimethyl-2-[N-(3-hydroxypropyl)-N-methylaminosulfonyl]-6-nitrobenzamide

N,N-Dimethyl-2-[N-(3-hydroxypropyl)-aminosulfonyl]-6-nitrobenzamide (1.37 g, 4.13 mmol) in 12 ml of DMF, was added dropwise to a suspension of 198 mg (4.13 mmol) of 50 % NaH in 3 ml of DMF in an atmosphere of $N_2$. When evolution of hydrogen was complete, a solution of methyl p-toluene sulfonate (0.794 g, 4.14 mmol) in DMF, 2 ml, was added. The clear deep yellow solution was stirred at 25° for 17 hours, then at 60° for 23 hours. The solution then was mixed with 175 ml of ethyl acetate and the precipitate that separated collected on a filter and washed with 50 ml of ethyl acetate. The combined filtrate and washings were extracted with 6 x 25 ml of saturated NaCl solution and the ethyl acetate evaporated to give 1.66 g of a clear yellow oil. Flash chromatography over E. Merck silica gel 60 (230 - 400 mesh) gave 0.49 g of a clear light yellow oil. This material was dissolved in 50 ml of $CHCl_3$, the solution extracted with 4 x 5 ml of 1N NaOH, then with 3 x 5 ml of saturated NaCl and dried over $MgSO_4$. After evaporation of the $CHCl_3$, the residue was dissolved in ethyl acetate and the

solution filtered to remove a trace of $MgSO_4$. Evaporation of the solvent gave 0.44 g of light yellow crystalline product, m.p., 91.5 - 92.5°.

**Claims**

1. 2-(substituted aminosulfonyl)-6-nitrobenzoic acids, esters or amides of the formula I

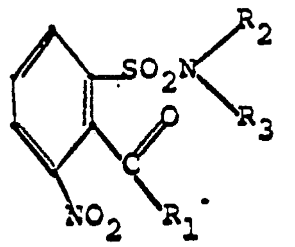

I

wherein
$R_1$ is hydroxy, alkoxy, hydroxyalkoxy, allyloxy, amino, monoalkylamino, dialkylamino, hydroxyalkylamino, di-(hydroxyalkyl, amino or allylamino and
$R_2$ and $R_3$ if they are taken separately are hydrogen, alkyl, hydroxyalkyl, allyl, aminoalkyl, monoalkylaminoalkyl, dialkylaminoalkyl, (hydroxyalkyl) amino-(lower alkyl), (hydroxyalkyl)-alkylamino-(lower alkyl) or di-(hydroxyalkyl)-amino-(lower alkyl) or
$R_2$ and $R_3$ form together with the nitrogen atom to which they are attached a heterocyclic ring radical selected from morpholino or 3-oxo-piperazino-1-yl or $R_4$-substituted 3-oxo-piperazino-1-yl wherein $R_4$ is alkyl or hydroxy alkyl,
provided that if both radicals $R_2$ and $R_3$ have the meaning of a hydrogen atom, then $R_1$ must not be an alkoxy group,
or salts of the compounds of formula I.

2. Compounds of formula I according to claim 1 wherein $R_1$ has the same meaning as defined in claim 1 and
$R_2$ and $R_3$ if they are taken separately have the same meaning as in claim 1, provided that least one of the radicals $R_2$ and $R_3$ is basic substituent selected from amino-(lower-alkyl), (lower alkyl)-amino-(lower alkyl), di-(loweralkyl)-amino-(lower alkyl), (hydroxyalkyl)-amino-(lower alkyl), (hydroxyalkyl)-alkylamino-(lower alkyl) or di-(hydroxyalkyl)-amino-(lower alkyl), or
salts of said compounds.

3. A compound according to claim 2 which is selected from methyl 2-[N-(2-dimethylaminoethyl)-aminosulfonyl]-6-nitrobenzoate hydrochloride; N,N-dimethyl-2-[N-(2-dimethylaminoethyl)-aminosulfonyl]-6-nitrobenzamide; 2-[N-(2-dimethylaminoethyl)-aminosulfonyl]-6-nitrobenzoic acid; N,N-dimethyl-2-[N-(2-dimethylaminomethyl)-N-(2-hydroxyethyl)-aminosulfonyl]-6-nitrobenzamide; and N,N-dimethyl-2- N-[2-(hydroxyethyl)-N-methylamino)-ethyl]-N-methylaminosulfonyl-6-nitrobenz-amide.

4. A compound of formula I according to claim 1, wherein
$R_1$ is alkoxy, hydroxyalkoxy, allyloxy, amino monoalkylamino, dialkylamino, hydroxyalkylamino, di-(hydroxyalkyl)-amino or allylamino and
$R_2$ and $R_3$ if they are taken separately are hydrogen, alkyl, hydroxyalkyl, allyl, or
$R_2$ and $R_3$ together with the nitrogen atom to which they are attached are a heterocyclic ring radical selected from morpholino, 3-oxo-piperazino-1-yl or $R_4$-substituted 3-oxo-piperazino-1-yl, wherein $R_4$ is alkyl or hydroxyalkyl,
provided that if both radicals $R_2$ and $R_3$ have the meaning of a hydrogen atom, then $R_1$ must not be an alkoxy group,
or salts of said compounds of formula I.

5. A compound according to claim 4, which is selected from methyl 2-[N-(2-hydroxyethyl)-N-methyl-aminosulfonyl]-6-nitrobenzoate; methyl 2-[N,N-di-(2-hydroxyethyl)-aminosulfonyl]-6-nitrobenzoate; methyl 6-nitro-2-[3-oxo-1-piperazinylsulfonyl]-benzoate; methyl 2-[N-morpholinosulfonyl]-6-nitrobenzoate; methyl 2-[N-(hydroxyethyl)-aminosulfonyl]-6-nitrobenzoate; N-(2-hydroxyethyl)-2-[N-(2-hydroxyethyl)-aminosulfonyl]-nitrobenzamide; allyl 2-[N-morpholinosulfonyl]-6-nitrobenzoate; N,N-dimethyl-2-[N-(2-hydroxyethyl)-aminosulfonyl]-6-nitrobenzamide; N,N-dimethyl-2-[N-(2-hydroxyethyl)-N-methylaminosulfonyl]-6-nitrobenzamide; and N,N- dimethyl-2-[N-(3-hydroxypropyl)-N-methylaminosulfonyl]-6-nitrobenzamide.

6. Process for the preparation of compounds of formula I

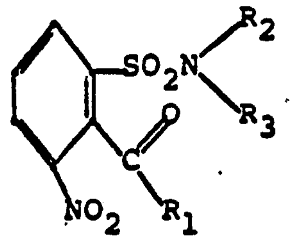

I

according to claim 1 or salts of the compounds of formula I

wherein

$R_1$, $R_2$ and $R_3$ have the same meaning as defined in claim 1, characterized in that a corresponding chlorosulfonyl compound of formule III

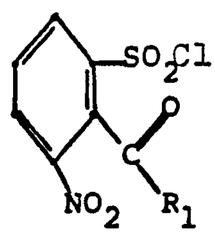

III

wherein

$R_1$ has the same meaning as defined in the compounds of formula I is reacted with ammonia, respectively an amine of formula II

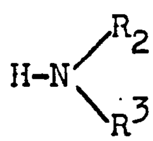

II

wherein

$R_2$ and $R_3$ have the same meaning as in formula I, preferably in an aprotic solvent, yielding the compounds of formula I which are isolated in the free form or as salts thereof

and that optionally resulting compounds of formula I wherein $R_1$ is hydroxy are esterified yielding corresponding compounds of formula I in which $R_1$ is alkoxy, hydroxyalkoxy or allyloxy which esters are isolated in the free form or as salts thereof

or that optionally resulting ester compounds of formula I wherein $R_1$ is alkoxy or hydroxyalkoxy are reacted with ammonia or an amine, preferably in a solvent, to yield corresponding compounds of formula I wherein $R_1$ is amino, monoalkylamino, dialkylamino, hydroxyalkylamino, di-(hydroxvalkyl)-amino or allylamino, which compounds of formula I are isolated the free form or as salts thereof.

7. Process for the preparation of compounds of formula Ia

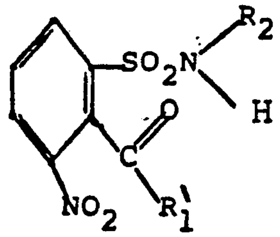

Ia

according to claim 1 or salts of the compounds of formula Ia

wherein

$R_1'$ is amino, monoalkylamino, dialkylamino, hydroxyalkylamino, di-(hydroxyalkyl)-amino or allylamino and

$R_2$ is hydrogen, alkyl, hydroxyalkyl, allyl, aminoalkyl, monoalkyl-aminoalkyl, dialkylaminoalkyl, (hydroxyalkyl)-amino-(lower alkyl), (hydroxyalkyl)-alkylamino-(lower alkyl) or di-(hydroxyalkyl)-amino-(lower alkyl),

characterized in that a compound of formula IV

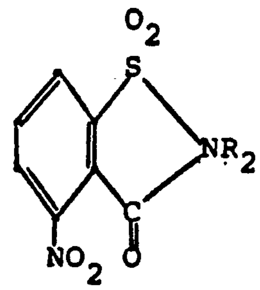

IV

wherein
$R_2$ has the same meaning as in formula Ia is reacted with ammonia or an amine of formula

$H-R_1'$

wherein $R_1'$ has the same meaning as in the compounds of formula Ia.

8. Process for the preparation of compounds of formula Ib

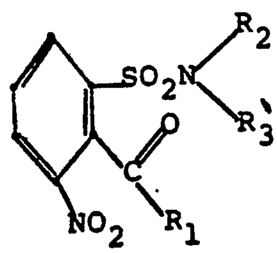

Ib

according to claim 1 or salts of the compounds of formula Ib,
wherein
$R_1$ has the same meaning as in claim 1 and
$R_2$ and $R_3'$ have the same meaning as the radicals $R_2$ and $R_3$ according to claim 1, except that the radical $R_3$ must not be a hydrogen atom,
characterized in that a compound of formula Ic

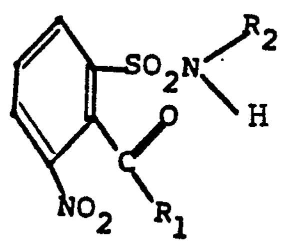

Ic

is alkylated introducing the radical $R_3'$ yielding the final product of formula Ib which is isolated in free form or as salts thereof.

9. Pharmaceutical composition for enhancing the therapeutic effect of radiation characterized in that it contains as pharmaceutically active ingredient a compound of formula I according to claim 1 or a pharmaceutically acceptable salt thereof.

10. Pharmaceutical composition according to claim 9 characterized in that it furthermore contains a pharmaceutically acceptable carrier material.

**Revendications**

1. Acides, esters ou amides 6-nitrobenzoïques 2-(aminosulfonyl substitué), de formule I

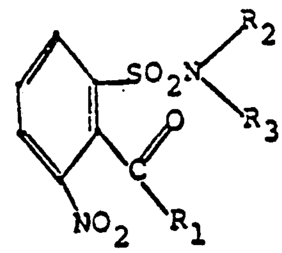

I

dans laquelle

$R_1$ est un groupe hydroxy, alcoxy, hydroxyalcoxy, allyloxy, amino monoalkylamino, dialkylamino, hydroxyalkylamino, di(hydroxyalkyl)amino ou allylamino, et

$R_2$ et $R_3$, s'ils sont pris séparément, sont un hydrogène, un groupe alkyle, hydroxyalkyle, allyle, aminoalkyle, monoalkylaminoalkyle, dialkylaminoalkyle, (hydroxyalkyl)amino(alkyle inférieur), (hydroxyalkyl)alkylamino(alkyle inférieur) ou di(hydroxyalkyl)amino(alkyle inférieur), ou

$R_2$ et $R_3$ forment conjointement avec l'atome d'azote auquel ils sont fixés un noyau hétérocyclique choisi parmi les groupes morpholino ou 3-oxo-pipérazinyle-1, ou 3-oxo-pipérazinyle-1-$R_4$ substitué où $R_4$ est un groupe alkyle ou hydroxyalkyle, à condition que, si les deux radicaux $R_2$ et $R_3$ représentent un atome d'hydrogène, $R_1$ ne soit pas un groupe alcoxy, ou les sels des composés de formule I.

2. Composés de formule I selon la revendication 1, dans laquelle $R_1$ a la même signification que celle définie dans la revendication 1 et

$R_2$ et $R_3$, s'ils sont pris séparément, ont la même signification que dans la revendication 1, à condition que l'un au moins des radicaux $R_2$ et $R_3$ soit un substituant basique choisi parmi les groupes amino(alkyle inférieur), (alkyl inférieur)amino(alkyle inférieur), di(alkyl inférieur)amino(alkyle inférieur), hydroxyalkylamino(alkyle inférieur), hydroxyalkylalkylamino(alkyle inférieur) ou di(hydroxyalkyl)amino(alkyle inférieur), ou les sels de ces composés.

3. Composé selon la revendication 2, qui est choisi parmi le chlorhydrate de 2-[N-(2-diméthylaminoéthyl)aminosulfonyl]-6-nitrobenzoate de méthyle; le N,N-diméthyl-2-[N-(2-diméthylaminoéthyl)aminosulfonyl]-6-nitrobenzamide; l'acide 2-[N-(2-diméthylaminoéthyl)aminosulfonyl]-6-nitrobenzoïque; le N,N-diméthyl-2-[N-(2-diméthylaminométhyl)-N-(2-hydroxyéthyl)aminosulfonyl]-6-nitrobenzamide; et le N,N-diméthyl-2-N-[2-(hydroxyéthyl)-N-méthylaminoéthyl]-N-méthylaminosulfonyl-6-nitrobenzamide.

4. Composé de formule I selon la revendication 1, dans lequel

$R_1$ est un groupe alcoxy, hydroxyalcoxy, allyloxy, amino, monoalkylamino, dialkylamino, hydroxyalkylamino, di(hydroxyalkyl)amino ou allylamino, et

$R_2$ et $R_3$, s'ils sont pris séparément, sont un hydrogène, un groupe alkyle, hydroxyalkyle, allyle, ou

$R_2$ et $R_3$ représentent, conjointement avec l'atome d'azote auxquels ils sont fixés, un noyau hétérocyclique choisi parmi les radicaux morpholino, 3-oxopipérazinyle-1 ou 3-oxopipérazinyle-1 $R_4$-substitué, dans lequel $R_4$ est un groupe alkyle ou hydroxyalkyle,

à condition que, si les deux radicaux $R_2$ et $R_3$ représentent un atome d'hydrogène, $R_1$ ne soit pas un groupe alcoxy, ou les sels des composés de formule I.

5. Composé selon la revendication 4, choisi parmi le 2-[N-(2-hydroxyéthyl)-N-méthylaminosulfonyl]-6-nitrobenzoate de méthyle; le 2-[N,N-di(2-hydroxyéthyl)aminosulfonyl]-6-nitrobenzoate de méthyle; le 6-nitro-2-[3-oxo-1-pipérazinylsulfonyl]benzoate de méthyle; le 2-[N-morpholinosulfonyl]-6-nitrobenzoate de méthyle; le 2-[N-(hydroxyéthyl)aminosulfonyl]-6-nitrobenzoate de méthyle; le N-(2-hydroxyéthyl)-2-[N-(2-hydroxyéthyl)aminosulfonyl]nitrobenzamide; le 2-[N-morpholinosulfonyl]-6-nitrobenzoate d'allyle; le N,N-diméthyl-2-[N-(2-hydroxyéthyl)aminosulfonyl]-6-nitrobenzamide; le N,N-diméthyl-2-[N-2-hydroxyéthyl)-N-méthylaminosulfonyl]-6-nitrobenzamide; et le N,N-diméthyl-2-[N-(3-hydroxypropyl)-N-méthylaminosulfonyl]-6-nitrobenzamide.

6. Procédé de préparation de composés de formule I selon la revendication 1, ou des sels des composés de formule I

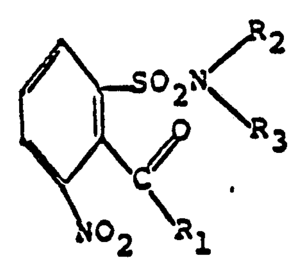

I

dans laquelle $R_1$, $R_2$ et $R_3$ ont les mêmes significations que celles définies dans la revendication 1, caractérisé en ce que l'on fait réagir un composé chlorosulfonylique de formule III

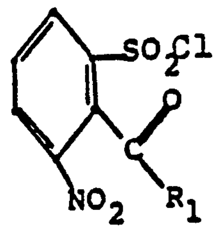

III

dans laquelle $R_1$ a la même signification que celle définie dans les composés de formule I, avec de l'ammoniac, respectivement une amine de formule II

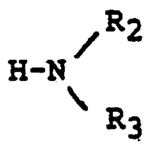

II

dans laquelle

$R_2$ et $R_3$ ont la même signification que dans la formule I, de préférence dans un solvant aprotique, ce qui donne les composés de formule I que l'on isole sous la forme libre ou sous forme de leurs sels, et en ce que l'on estérifie éventuellement les composés résultants de formule I, dans laquelle $R_1$ est un groupe hydroxy, ce qui donne les composés correspondants de formule I, dans laquelle $R_1$ est un groupe alcoxy, hydroxyalcoxy ou allyloxy, ces esters étant isolés sous la forme libre ou sous forme de leurs sels, ou en ce que l'on fait réagir éventuellement les composés esters résultants de formule I, dans laquelle $R_1$ est un groupe alcoxy ou hydroxyalcoxy, avec de l'ammoniac ou une amine, de préférence dans un solvant, pour obtenir les composés correspondants de formule I, dans laquelle $R_1$ est un groupe amino, monoalkylamino, dialkylamino, hydroxyalkylamino, di(hydroxyalkyl)amino ou allylamino, ces composés de formule I étant isolés sous la forme libre ou sous forme de leurs sels.

7. Procédé de préparation de composés de formule Ia

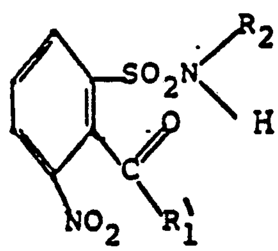

Ia

selon la revendication 1, ou des sels des composés de formule Ia, dans laquelle

$R_1'$ est un groupe amino, monoalkylamino, dialkylamino, hydroxyalkylamino, di(hydroxyalkyl)amino ou allylamino, et $R_2$ est un hydrogène, un groupe alkyle, hydroxyalkyle, allyle, aminoalkyle, monoalkylaminoalkyle, dialkylaminoalkyle, hydroxyalkylamino(alkyle inférieur), hydroxyalkylalkylamino(alkyle inférieur) ou di(hydroxyalkyl)amino(alkyle inférieur),

caractérisé en ce que on fait réagir un composé de formule IV

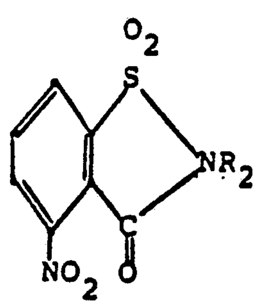

IV

dans laquelle $R_2$ a la même signification que dans la formule Ia, avec de l'ammoniac ou une amine de formule

$H\text{-}R_1'$

dans laquelle $R_1'$ a la même signification que dans les composés de formule Ia.

8. Procédé de préparation de composés de formule Ib

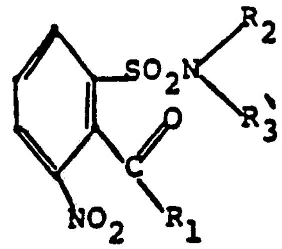

Ib

selon la revendication 1, ou de sels des composés de formule Ib, dans laquelle

$R_1$ a la même signification que dans la revendication 1 et

$R_2$ et $R_3'$ ont la même signification que les radicaux $R_2$ et

$R_3$ selon la revendication 1, sauf que le radical $R_3'$ ne doit pas être un atome d'hydrogène,

caractérisé en ce que l'on alkyle un composé de formule Ic en introduisant le radical $R_3$, ce qui donne le produit final de formule Ib, que l'on isole sous la forme libre ou sous forme de sels.

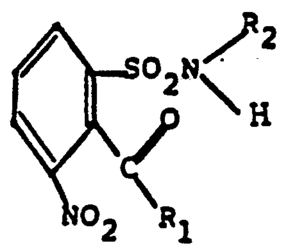

Ic

9. Composition pharmaceutique pour augmenter l'effet thérapeutique d'un rayonnement, caractérisée en ce qu'elle contient, comme substance pharmaceutiquement active, un composé de formule I selon la revendication 1 ou un sel pharmaceutiquement acceptable de ce composé.

10. Composition pharmaceutique selon la revendication 9, caractérisée en ce qu'elle contient en outre un véhicule pharmaceutiquement acceptable.

**Patentansprüche**

1. 2-(substituiertes Aminosulfonyl)-6-nitrobenzoesäuren, -ester oder -amide der Formel I

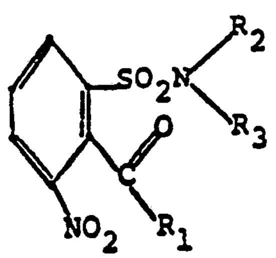

I

worin $R_1$ Hydroxy, Alkoxy, Hydroxyalkoxy, Allyloxy, Amino, Monoalkylamino, Dialkylamino, Hydroxyalkylamino, Di(hydroxyalkyl)amino oder Allylamino ist, und

$R_2$ und $R_3$, falls getrennt genommen, Wasserstoff, Alkyl, Hydroxyalkyl, Allyl, Aminoalkyl, Monoalkylaminoalkyl, Dialkylaminoalkyl, (Hydroxyalkyl)amino(niedrigalkyl), (Hydroxyalkyl)alkylamino(niedrigalkyl) oder Di(hydroxyalkyl)amino(niedrigalkyl) sind, oder

$R_2$ und $R_3$ zusammen mit dem Stickstoffatom, an das sie gebunden sind, einen aus Morpholino oder 3-Oxo-piperazino-1-yl oder $R_4$-substituiertern 3-Oxo-piperazino-1-yl ausgewählten heterocyclischen Ringrest bilden, worin $R_4$ Alkyl oder Hydroxyalkyl ist,

mit der Maßgabe, daß, falls beide Reste $R_2$ und $R_3$ ein Wasserstoffatom bedeuten, $R_1$ keine Alkoxygruppe sein darf, oder Salze der Verbindungen der Formel I.

2. Verbindung der Formel I nach Anspruch 1, worin $R_1$ die gleiche Bedeutung hat, wie in Anspruch 1 angegeben, und

$R_2$ und $R_3$, falls getrennt genommen, die gleiche Bedeutung haben, wie in Anspruch 1, mit der Maßgabe, daß wenigstens einer der Reste $R_2$ und $R_3$ ein aus Amino(niedrigalkyl), (Niedrigalkyl)amino(niedrigalkyl), Di(niedrigalkyl)amino(niedrigalkyl), (Hydroxyalkyl)amino(niedrigalkyl), (Hydroxyalkyl)alkylamino(niedrigalkyl) oder Di(hydroxyalkyl)amino(niedrigalkyl) ausgewählter Basissubstituent ist, oder Salze dieser Verbindungen.

3. Verbindung nach Anspruch 2, ausgewählt aus Methyl-2-[N-(2-dimethylaminoethyl)aminosulfonyl]-6-nitrobenzoat-hydrochlorid; N,N-Dimethyl-2-[N-(2-dimethylaminoethyl)aminosulfonyl]-6-nitrobenzamid; 2-[N-(2-Dimethylaminoethyl)aminosulfonyl]-6-nitrobenzoesäure; N,N-Dimethyl-2-[N-(2-dimethylaminomethyl)-N-(2-hydroxyethyl)aminosulfonyl]-6-nitrobenzamid; und N,N-Dimethyl-2-N-[2-(hydroxyethyl)-N-methylamino)-ethyl]-N-methylaminosulfonyl-6-nitrobenzamid.

4. Verbindung der Formel I nach Anspruch 1, worin

$R_1$ Alkoxy, Hydroxyalkoxy, Allyloxy, Aminomonoalkylamino, Dialkylamino, Hydroxyalkylamino, Di(hydroxyalkyl)amino oder Allylamino ist, und

$R_2$ und $R_3$, falls getrennt genommen, Wasserstoff, Alkyl, Hydroxyalkyl, Allyl sind, oder

$R_2$ und $R_3$ zusammen mit dem Stickstoffatom, an das sie gebunden sind, ein aus Morpholino, 3-Oxo-piperazino-1-yl oder $R_4$-substituiertem 3-Oxo-piperazino-1-yl, worin $R_4$ Alkyl oder Hydroxyalkyl ist, ausgewählter heterocyclischer Ringrest sind,

mit der Maßgabe, daß, falls beide Reste $R_2$ und $R_3$ ein Wasserstoffatom bedeuten, $R_1$ keine Alkoxygruppe sein darf, oder Salze der Verbindungen der Formel I.

5. Verbindung nach Anspruch 4, ausgewählt aus Methyl-2-[N-(2-hydroxyethyl)-N-methylaminosulfonyl]-6-nitrobenzoat; Methyl-2-[N,N-di(2-hydroxyethyl)aminosulfonyl]-6-nitrobenzoat; Methyl-6-nitro-2-[3-oxo-1-piperazinylsulfonyl]benzoat; Methyl-2-[N-morpholinosulfonyl]-6-nitrobenzoat; Methyl-2-[N-(hydroxyethyl)aminosulfonyl]-6-nitrobenzoat; N-(2-Hydroxyethyl)-2-[N-(2-hydroxyethyl)aminosulfonyl]nitrobenzamid; Allyl-2-[N-morpholinosulfonyl]-6-nitrobenzoat; N,N-Dimethyl-2-[N-(2-hydroxyethyl)aminosulfonyl]-6-nitrobenzamid; N,N-Dimethyl-2-[N-2-hydroxyethyl)-N-methylaminosulfonyl]-6-nitrobenzamid; und N,N-Dimethyl-2-[N-(3-hydroxypropyl)-N-methylaminosulfonyl]-6-nitrobenzamid.

6. Verfahren zur Herstellung von Verbindungen der Formel I

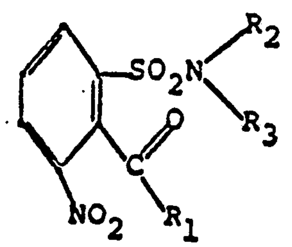

I

nach Anspruch 1 oder von Salzen der Verbindungen der Formel I, worin $R_1$, $R_2$ und $R_3$ die gleiche Bedeutung haben, wie in Anspruch 1 angegeben, dadurch gekennzeichnet, daß eine entsprechende Chlorosulfonylverbindung der Formel III

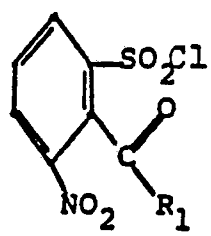

III

worin $R_1$ die gleiche Bedeutung hat, wie für die Verbindungen der Formel I angegeben, mit Ammoniak oder einem Amin der Formel II

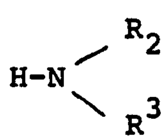

II

worin $R_2$ und $R_3$ die gleiche Bedeutung haben, wie in Formel I, vorzugsweise in einem aprotischen Lösungsmittel unter Erhalt der Verbindungen der Formel I, die in der freien Form oder als Salze davon isoliert werden, reagiert wird, und daß gegebenenfalls resultierende Verbindungen der Formel I, worin $R_1$ Hydroxy ist, unter Erhalt der entsprechenden Verbindungen der Formel I, worin $R_1$ Alkoxy, Hydroxyalkoxy oder Allyloxy ist, verestert werden, wobei die Ester in der freien Form oder als Salze davon isoliert werden,

oder daß gegebenenfalls resultierende Esterverbindungen der Formel I, worin $R_1$ Alkoxy oder Hydroxyalkoxy ist, mit Ammoniak oder einem Amin vorzugsweise in einem Lösungsmittel unter Erhalt der entsprechenden Verbindungen der Formel I, worin $R_1$ Amino, Monoalkylamino, Dialkylamino, Hydroxyalkylamino, Di(hydroxyalkyl)amino oder Allylamino ist, reagiert werden, wobei die Verbindungen der Formel I in der freien Form oder als Salze davon isoliert werden.

7. Verfahren zur Herstellung von Verbindungen der Formel Ia nach Anspruch 1 oder von Salzen der Verbindungen der Formel Ia,

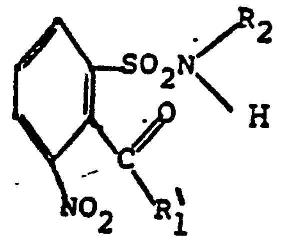

Ia

worin $R_1'$ Amino, Monoalkylamino, Dialkylamino, Hydroxyalkylamino, Di(hydroxyalkyl)amino oder Allylamino ist, und

$R_2$ Wasserstoff, Alkyl, Hydroxyalkyl, Allyl, Aminoalkyl, Monoalkylaminoalkyl, Dialkylaminoalkyl, (Hydroxyalkyl)amino-(niedrigalkyl), (Hydroxyalkyl)alkylamino(niedrigalkyl) oder Di(hydroxyalkyl)amino(niedrigalkyl) ist, dadurch gekennzeichnet, daß in einer Verbindung der Formel IV

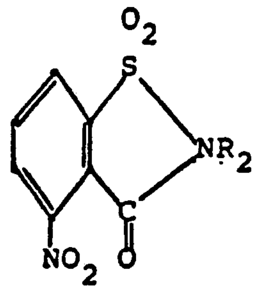

IV

worin $R_2$ die gleiche Bedeutung hat wie in Formel Ia, mit Ammoniak oder einem Amin der Formel

$H\text{-}R_1'$

worin $R_1'$ die gleiche Bedeutung hat wie in den Verbindungen der Formel Ia, reagiert wird.

8. Verfahren zur Herstellung von Verbindungen der Formel Ib

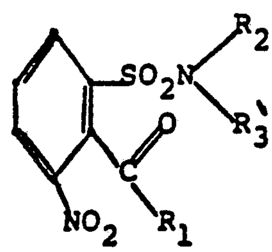

Ib

nach Anspruch 1 oder vcn Salzen der Verbindungen der Formel Ib,

worin $R_1$ die gleiche Bedeutung hat, wie in Anspruch 1, und

$R_2$ und $R_3'$ die gleiche Bedeutung haben, wie die Reste $R_2$ und $R_3$ gemäß Anspruch 1, außer daß der Rest $R_3'$ kein Wasserstoffatom sein darf,

dadurch gekennzeichnet, daß eine Verbindung der Formel Ic

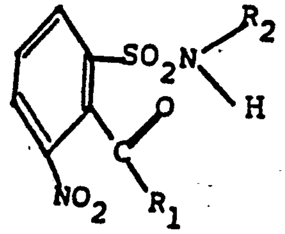

Ic

unter Einführung des Restes $R_3'$ und Erhalt des Endprodukts der Formel Ib, das in freier Form oder als Salz davon isoliert wird, alkyliert wird.

9. Pharmazeutische Zusammensetzung zur Verstärkung des therapeutischen Effekts von Strahlung, dadurch gekennzeichnet, daß sie als pharmazeutisch wirksamen Bestandteil eine Verbindung der Formel I gemäß Anspruch 1 oder ein pharmazeutisch annehmbares Salz davon enthält.

10. Pharmazeutische Zusammensetzung nach Anspruch 9, dadurch gekennzeichnet, daß sie weiterhin ein pharmazeutisch annehmbares Trägermaterial enthält.